# EUROPEAN PATENT APPLICATION

(11) **EP 3 053 506 A1**
(43) Date of publication of application: **10.08.2016**
(21) Application number: 15154180.2
(22) Date of filing: 06.02.2015
(51) Int. Cl.: A61B 1/00, A61B 1/303, A61B 1/32

(54) **Intravaginal camera**

(71) Applicant: Qioptiq Photonics GmbH & Co. KG, 37081 Göttingen (DE)
(72) Inventor: Gotsch, Klaus, 81241 Muenchen (DE); Partheymueller, Ulrich, Holzkirchen (DE); Mumm, Bernhard, 82291 Mammendorf (DE); Balbach, Thomas, 85777 Fahrenzhausen (DE)
(74) Representative: Brookes Batchellor LLP

(57) **Abstract**

An intravaginal imaging device is disclosed. The imaging device is housed within an elongated housing having a proximal end and a distal end. The distal end includes a sealing endcap window, an objective disposed behind the window having an auto focus mechanism configured to focus on a target, an image detector disposed behind the objective configured to detect an image from the objective, and a light source disposed behind the window configured to illuminate the target. A connecting portion in communication with the image detector is disposed at the proximal end. The imaging device includes attachment means configured to attach a substantially ring shaped vagina expander to the distal end of the elongated housing. The connecting portion is configured to convey an image from the image detector to an external device.

## Description

### FIELD OF THE INVENTION

The present invention relates to medical imaging, and more particularly, is related to an intravaginal camera.

### BACKGROUND OF THE INVENTION

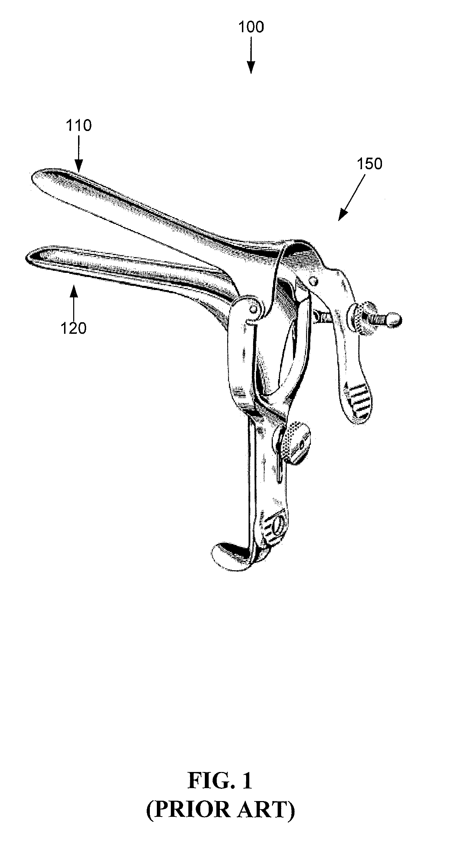

Colposcopy is a medical diagnostic procedure to examine an illuminated, magnified view of the cervix and the tissues o f the vagina and vulva. There are known methods and instrumentation used to observe changes of the cervix mucosa. Inspection of the cervix has generally been performed by insertion of medical devices inside in the vagina. The medical devices, such as a speculum 100, as shown in FIG. 1, separate the soft tissue of the vaginal walls and provide a direct optical path to the cervix for an external optical device, such as a colposcope. The speculum 100 also provides a path for an external light source to illuminate the cervix.

However, use of a speculum 100 has undesirable qualities, such as excessive expansion of the vagina wall throughout the entire length of the vagina, rather than just in the vicinity of the cervix. The result is unnecessary deformation of the vagina and cervix region, as well as unnecessary discomfort for the patient. In addition, the colposcope and additional standalone examination unit are expensive, and may not be practically used in combination with other diagnostic tools, such as ultrasound systems.

For some intravaginal cameras, focus adjustment is performed by manually adjusting the distance between the lens and sensor. But this method is not convenient for intravaginal applications. Some intravaginal cameras have a small numerical aperture (NA) that can provide a large depth of field (DOF) to replace focus adjustment. But small NA optical systems are generally unsuitable to provide high resolution and may increase the luminous flux.

A liquid lens camera, for example, an intraoral camera as disclosed in patent applications such as EP 2161607 A1, EP 1780757 A1, and PCT/CN2008/001900, generally includes a first liquid and a second liquid of equal density sandwiched between two transparent windows in a conical vessel. The first liquid is generally conductive, while the second liquid is generally insulating. A variable voltage can be selectively applied to electrodes in electrical communication with the conductive liquid. The interface between the first and second liquid changes its shape depending on the voltage applied across the conical structure. In this way, the liquid lens can attain the desired refraction power by means of changing the voltage applied on the electrodes. The variation of voltage leads to a change of curvature of the liquid-liquid interface, which in turn leads to a change of the focal length of the lens. However, several factors including the orientation of the camera in relation to the housing and potential buildup of mucus around the lenses make the intraoral camera unsuitable for intravaginal use. Therefore, there is a need in the industry to address some of the abovementioned shortcomings.

### SUMMARY OF THE INVENTION

Embodiments of the present invention provide an intravaginal camera. Briefly described, the present invention is directed to an intravaginal imaging device. The imaging device is housed within an elongated housing having a proximal end and a distal end. The distal end includes a sealing endcap window, an objective disposed behind the window having an auto focus mechanism configured to focus on a target, an image detector disposed behind the objective configured to detect an image from the objective, and a light source configured to illuminate the target. A connecting portion in communication with the image detector is disposed at the proximal end. The imaging device includes attachment means configured to attach a substantially ring shaped vagina expander to the distal end of the elongated housing. The connecting portion is configured to convey an image from the image detector to an external device.

Other systems, methods and features of the present invention will be or become apparent to one having ordinary skill in the art upon examining the following drawings and detailed description. It is intended that all such additional systems, methods, and features be included in this description, be within the scope of the present invention and protected by the accompanying claims.

### BRIEF DESCRIPTION OF THE DRAWINGS

The accompanying drawings are included to provide a further understanding of the invention, and are incorporated in and constitute a part of this specification. The components in the drawings are not necessarily to scale, emphasis instead being placed upon clearly illustrating the principles of the present invention. The drawings illustrate embodiments of the invention and, together with the description, serve to explain the principals of the invention.
FIG. 1 is a schematic diagram of a prior art speculum.
FIG. 2 is a schematic cutaway diagram showing a first exemplary embodiment of an intravaginal camera.
FIG. 3 is a detail of the distal end of the intravaginal camera of FIG. 2.

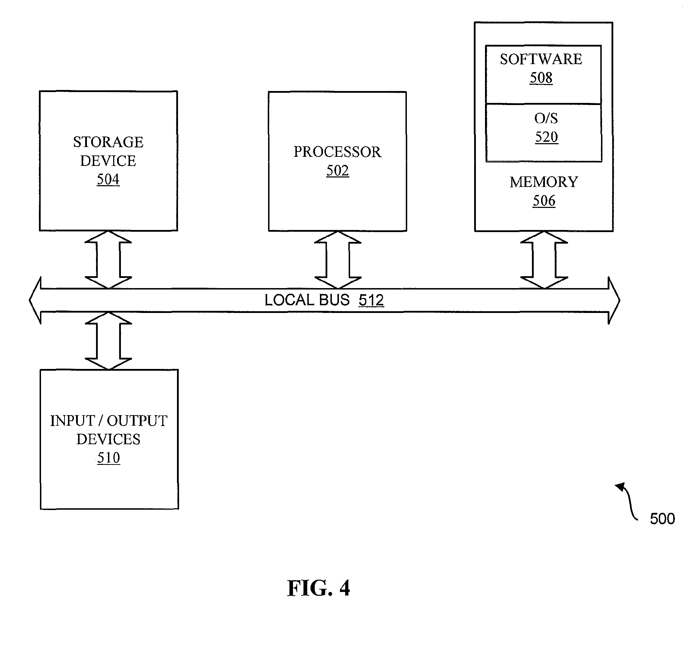

FIG. 4 is a schematic diagram illustrating an example of a system for executing functionality of the present invention.

### DETAILED DESCRIPTION

Reference will now be made in detail to embodiments of the present invention, examples of which are illustrated in the accompanying drawings. Wherever possible, the same reference numbers are used in the drawings and the description to refer to the same or like parts.

Exemplary embodiments of an intravaginal camera are intended to perform intravaginal acquisition, cervix acquisition, and the like. It is therefore desirable for a lens module of the camera to have a large depth of field (DOF) and wide field of view (FOV) in a large range of working distances, for example, from under 1 mm to infinity. The camera may be used in a large working distance range with a big DOF. The intravaginal camera may focus at a far distance, for example, when positioned at the entrance of the vagina, or at a near distance, for example, when positioned adjacent to the cervix. Focus adjustment is used to provide appropriate image quality.

Generally, a first embodiment of an intravaginal camera includes a housing enclosing an illumination system, a lens system, attachment means for a vagina expander, and electrical components. The illumination system is used to provide enough light to illuminate the cervix and vagina interior. Polarized illumination may be used to limit reflections. For example, one or more white light emitting diodes (LEDs) may be used in the illumination system, which may be preferable due to small size, long lifetime and high luminous flux. Similarly, illumination in one or more specific wavelengths or bands of wavelengths may be used to directly or indirectly illuminate the target, for example via a fluorescence effect. For example, the illumination system may include LEDs with different colors, such as red/green/blue or other color combinations in order to produce a better tissue image, or to highlight or discern certain features in the image.

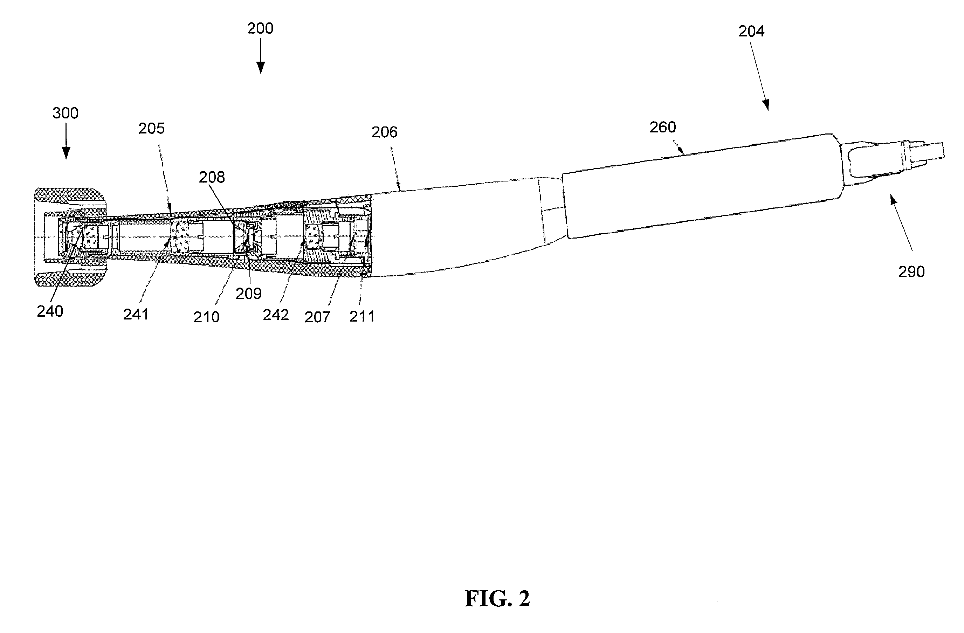

A first embodiment of the invention is explained in detail with reference to FIG. 2, which shows the intravaginal camera 200 in a schematic half-section. The elongated housing 206 houses optics and electronics. The optics may include a series of fixed lens groups 240, 241, 242 which map a target object (not shown), such as the cervix of a patient, on an image detector 207. The fixed lens groups 240, 241, 242 may be formed of optical glass. In alternative embodiments, one or more of the fixed lens groups 240, 241, 242 may be formed of sapphire, or other appropriate materials. While three fixed lens groups are shown in the first embodiment, alternative embodiments may include one, two, four or more fixed lens groups.

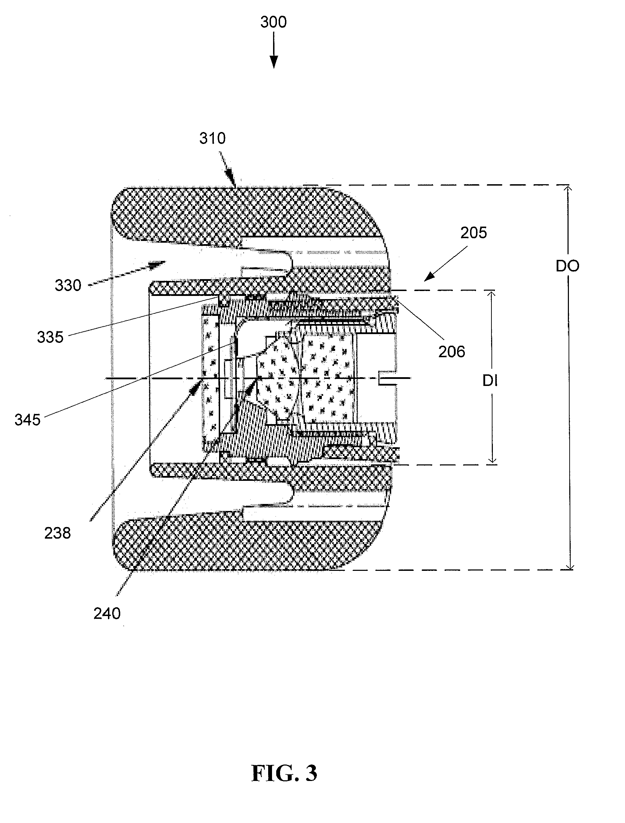

Here, the fixed lens groups 240, 241, and 242 are positioned between the target object (not shown) and the image detector 207. The first fixed lens group 240 is located behind an ingress window 238 (FIG. 3) at the distal end 205 of the elongated housing 206. The first fixed lens group 240 conveys an image to a second fixed lens group 241. Likewise, the second fixed lens group 241 and the third fixed lens group 242 transmit the image to the image detector 207.

As shown in FIG. 2, each of the three fixed lens groups 240, 241, 242 has two fixed lenses. However, in alternative embodiments there may be more or fewer fixed lens groups, and each fixed lens group may include, one, two, three or more lenses.

A variable lens 208 is positioned between the second fixed lens 241 and the third fixed lens 242. In alternative embodiments, the variable lens 208 may be positioned elsewhere with respect to the fixed lens groups, such as behind the third lens group 242. The liquid lens is preferably located near the aperture 210. The variable lens 208 under the first embodiment is a liquid lens. However, other variable lenses are possible in alternative embodiments. For example, rather than a liquid lens, one or more conventional solid lenses may be configured to be movable along an optical axis of the distal end 205 of the elongated housing 206, behaving as a classical focusing system. The fixed lens groups 240, 241, 242, the variable lens 208 and the ingress window 238 share a common optical axis. In general, the illumination provided by the illumination system is aligned with the common optical axis.

The imaging characteristics of the variable liquid lens 208 are controllable with electrical voltage as provided to electrodes (not shown) via an electrical lead 209 from an electrical controller 211. The electrical controller 211 may be housed within the elongated housing 206, as shown, or may be external to the elongated housing in alternative embodiments.

Further, the elongated housing 206 of the intravaginal camera 200 includes a variable aperture 210, the diameter of which is adjustable in a specified ratio to the focal position. The variable aperture 210 may be implemented as a liquid crystal panel (LCD), which is controlled by a further electrical voltage via an additional electrical connection (not shown). In alternative embodiments a mechanical aperture may be used.

The image detector 207 may be trained with the controller 211 to control the image sharpness. The controller 211 may further control the voltage for the variable liquid lens 208 via the electrical lead 209. The processor may be implemented as a computer, as described further below. An image at the image detector 207 may further include a scale configured to indicate a size of a region of interest. For example, a scale may be blended with the image at the image detector 207, or the controller 211 may superimpose upon the image of the image detector. The scale may be faded into the image at a desired intensity.

A sheath 260 may surround the proximal end 204 of the elongated housing 206. The center axis of the proximal end 204 of the elongated housing 206 may not be aligned with the center axis of the distal end 205 of the elongated housing 206. As a result, merely rotating the proximal end 204 around its axis outside the vagina may reposition the distal end 205 near the cervix, for example, allowing the user to more easily locate the portio of the cervix.

A connecting portion 290 may be located at the proximal end 204 of the elongated housing. The connecting portion may provide physical connections, for example, electrical connections and or gas/fluid connections, for example, for expanding/deflating the vagina expander 310 (FIG. 3). The connecting portion may further include means for wireless connection, for example, WiFi or BlueTooth wireless connections.

The elongated housing 206 may be formed of a biocompatible plastic, such as a medical grade biocompatible plastic. Alternatively, other materials may be used for the housing that are suitable for sterilization, such as, but not limited to silicone, latex, or metal such as medical grade titanium.

Under the first embodiment, the elongated housing 206 is approximately 293mm in length, has a maximum diameter of approximately 25.7mm, and a minimum diameter at the distal end 205 of approximately 12.7mm (without the vagina expander 310). Of course, these dimensions are provided as a non-limiting example, and dimensions of alternative embodiments may vary significantly.

FIG. 3 shows a detail 300 of the distal end 205 of the elongated housing 206. A transparent ingress window 238 may serve as an end cap to the distal end 205 of the elongated housing 206, providing both an optical ingress into the camera 200, as well as a fluid seal to the interior of the camera 200. Illumination means 345, such as one or more white LEDs may be disposed behind the ingress window 238 to provide illumination to the target of the camera 200. The illumination levels of the illumination means 345 may be controlled by the electrical controller 211 located within the elongated housing 206 or a remote controller (not shown) located remotely to the elongated housing 206.

A vagina expander 310 may be removably fastened to the distal end 205 of the elongated housing 206. The vagina expander 310 may be configured as a generally ring shaped tube structure substantially surrounding the distal end 205 of the distal end 205 of the elongated housing 206, such that the soft tissue is held apart to allow a clear field of view in front of the distal end 205 of the elongated housing 206. The optical path of the intravaginal camera 200 passes through an aperture in the vagina expander 310.

The vagina expander 310 may be removably attached to the distal end 205 of the elongated housing 206 by one or more of several attachment means 335, for example, a threaded attachment, a tongue in groove attachment, a friction fit attachment where the vagina expander is attached by friction to an otherwise smooth external surface of the distal end 205 of the elongated housing 206, or other means familiar to persons having ordinary skill in the art. The attachment means 335 are generally disposed at an inner diameter D1 of the vagina expander, and mate with corresponding means at or near the distal end 205 of the elongated housing 206, in particular, around an outer diameter at or near of the distal end 205 of the elongated housing 206.

Under the first embodiment, the vagina expander 310 includes a mucus trap 330, formed as a recessed region in a forward facing portion of the vagina expander. The mucus trap 330 may extend entirely around the distal end 205 of the elongated housing 206, such that mucus collects within the recess rather than collecting in front of the ingress window 230, thereby keeping the optical and/or illumination path of the intravaginal camera 200 unobstructed and unobscured.

While different embodiments of the vagina expander 310 may have different fixed sizes, the interior diameter DI is substantially the same, being configured to mate to the distal end 205 of the elongated housing 206. However, in alternate embodiments the vagina expander 310 may have differently sized outer diameters DO, for example, but not limited to in the range of 10 mm up to 50 mm.

While the intravaginal camera 200 distal end 205 of the elongated housing 206 includes means for connecting to an optional vagina expander 310, the intravaginal camera 200 may be operated without a vagina expander 310. For example, the intravaginal camera 200 may be used in conjunction with a speculum 100 (FIG. 1), where the intravaginal camera 200 is inserted within the jaws 150 (FIG. 1) of the speculum 100 (FIG. 1), and then positioned within the spacers 110, 120 (FIG. 1) as needed to obtain access to the desired target, for example, the cervix. It should be noted that when used in this fashion, the intravaginal camera 200 is preferably not attached to the speculum 100 (FIG. 1), but rather used independently. Therefore, the use of the term "vagina expander" within this disclosure and the claims should not be interpreted to mean a speculum.

Alternatively, the intravaginal camera 200 may be used in combination with a speculum 100 using a suitable holder provided by the speculum 100 or/and intravaginal camera 200 so as to affix the intravaginal camera 200 to the speculum 100, or provide a movable connection so that the intravaginal camera 200 is moveable along the speculum, for example, along a rail or slot.

In a second embodiment the intravaginal camera 200 may include a variably sized vagina expander 310. For example, the intravaginal camera 200 may include means for expanding or contracting the outer diameter DO of the vagina expander 310. Such expansion/contraction means may include inflation/deflation of gasses and/or fluids in an expanding/contracting tube within the vagina expander 310. Control of the amount of expansion/contraction may be controlled by the controller 211 located within the housing 206, or external to the housing 206. In alternative embodiments, the vagina expander 310 may be implemented as a disposable or cleanable tube or cover pulled over the elongated housing 206.

As previously mentioned, the controller 211 (FIG. 2) for executing the functionality described in detail above may be a computer, an example of which is shown in the schematic diagram of FIG. 5. Such functionality may be related to the systems described above, for example, the illumination System, the focus or autofocus system, the auto-inflation system, and other such systems that may be electronically controlled. The system 500 contains a processor 502, a storage device 504, a memory 506 having software 508 stored therein that defines the abovementioned functionality, input and output (I/O) devices 510 (or peripherals), and a local bus, or local interface 512 allowing for communication within the system 500. The local interface 512 can be, for example but not limited to, one or more buses or other wired or wireless connections, as is known in the art. The local interface 512 may have additional elements, which are omitted for simplicity, such as controllers, buffers (caches), drivers, repeaters, and receivers, to enable communications. Further, the local interface 512 may include address, control, and/or data connections to enable appropriate communications among the aforementioned components.

The processor 502 is a hardware device for executing software, particularly that stored in the memory 506. The processor 502 can be any custom made or commercially available single core or multi-core processor, a central processing unit (CPU), an auxiliary processor among several processors associated with the present system 500, a semiconductor based microprocessor (in the form of a microchip or chip set), a macroprocessor, or generally any device for executing software instructions.

The memory 506 can include any one or combination of volatile memory elements (e.g., random access memory (RAM, such as DRAM, SRAM, SDRAM, *etc*.)) and nonvolatile memory elements (e.g., ROM, hard drive, tape, CDROM, etc.). Moreover, the memory 506 may incorporate electronic, magnetic, optical, and/or other types of storage media. Note that the memory 506 can have a distributed architecture, where various components are situated remotely from one another, but can be accessed by the processor 502.

The software 508 defines functionality performed by the system 500, in accordance with the present invention. The software 508 in the memory 506 may include one or more separate programs, each of which contains an ordered listing of executable instructions for implementing logical functions of the system 500, as described below. The memory 506 may contain an operating system (O/S) 520. The operating system essentially controls the execution of programs within the system 500 and provides scheduling, input-output control, file and data management, memory management, and communication control and related services.

The I/O devices 510 may include input devices, for example but not limited to, a keyboard, mouse, scanner, microphone, *etc.* An input device may include a switch or actuator configured to start the autofocus procedure, for example, implemented as a switch on the housing, an external wired or wireless footswitch or by a keyboard, keypad, touchscreen, or other mechanism. Furthermore, the I/O devices 510 may also include output devices, for example but not limited to, a display, et cetera, which may be connected via a USB connector, or the illumination means 345 (FIG. 3). Finally, the I/O devices 510 may further include devices that communicate via both inputs and outputs, for instance but not limited to, a wireless communication system, a radio frequency (RF) or other transceiver, a telephonic interface, a bridge, a router, or other device.

When the system 500 is in operation, the processor 502 is configured to execute the software 508 stored within the memory 506, to communicate data to and from the memory 506, and to generally control operations of the system 500 pursuant to the software 508, as explained above.

When the functionality of the system 500 is in operation, the processor 502 is configured to execute the software 508 stored within the memory 506, to communicate data to and from the memory 506, and to generally control operations of the system 500 pursuant to the software 508. The operating system 520 is read by the processor 502, perhaps buffered within the processor 502, and then executed.

When the system 500 is implemented in software 508, it should be noted that instructions for implementing the system 500 can be stored on any computer-readable medium for use by or in connection with any computer-related device, system, or method. Such a computer-readable medium may, in some embodiments, correspond to either or both the memory 506 or the storage device 504. In the context of this document, a computer-readable medium is an electronic, magnetic, optical, or other physical device or means that can contain or store a computer program for use by or in connection with a computer-related device, system, or method. Instructions for implementing the system can be embodied in any computer-readable medium for use by or in connection with the processor or other such instruction execution system, apparatus, or device. Although the processor 502 has been mentioned by way of example, such instruction execution system, apparatus, or device may, in some embodiments, be any computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the processor or other such instruction execution system, apparatus, or device.

Such a computer-readable medium can be, for example but not limited to, an electronic, magnetic, optical, electromagnetic, infrared, or semiconductor system, apparatus, device, or propagation medium. More specific examples (a nonexhaustive list) of the computer-readable medium would include the following: an electrical connection (electronic) having one or more wires, a portable computer diskette (magnetic), a random access memory (RAM) (electronic), a read-only memory (ROM) (electronic), an erasable programmable read-only memory (EPROM, EEPROM, or Flash memory) (electronic), an optical fiber (optical), and a portable compact disc read-only memory (CDROM) (optical). Note that the computer-readable medium could even be paper or another suitable medium upon which the program is printed, as the program can be electronically captured, via for instance optical scanning of the paper or other medium, then compiled, interpreted or otherwise processed in a suitable manner if necessary, and then stored in a computer memory.

In an alternative embodiment, where the system 500 is implemented in hardware, the system 500 can be implemented with any or a combination of the following technologies, which are each well known in the art: a discreet logic circuit(s) having logic gates for implementing logic functions upon data signals, an application specific integrated circuit (ASIC) having appropriate combinational logic gates, a programmable gate array(s) (PGA), a field programmable gate array (FPGA), etc.

The above described embodiments of an intravaginal camera may be used for cervix inspection inside in the vagina with or without a speculum, because of the ergonomic shape including the vagina expander or inflatable bellow. This assists in observing the cervix regarding changes of the cervix mucosa. Advantages include lower costs compared with traditional colposcopy equipment and procedures, and no mandatory usage of a speculum, which is more comfortable and convenient for the patient. The intravaginal camera may be used in combination with ultrasound systems, for example via USB interface, providing a direct view and LED illumination on the region of interest (ROI) based on intravaginal use. The intravaginal camera may provide a digital image for recording and data transfer. In addition the ergonomic shape provides easy handling and avoids contamination of the internal optics.

In summary, it will be apparent to those skilled in the art that various modifications and variations can be made to the structure of the present invention without departing from the scope or spirit of the invention. In view of the foregoing, it is intended that the present invention cover modifications and variations of this invention provided they fall within the scope of the following claims and their equivalents.

## Claims

1. An intravaginal imaging device, comprising:
an elongated housing (206) comprising:
a proximal end (204);
a distal end (205), further comprising:
an endcap window configured to seal the distal end;
an objective disposed behind the window comprising a focus mechanism configured to focus on a target;
an image detector (207) disposed behind the objective configured to detect an image from the objective;
a light source configured to illuminate the target;
a connecting portion (290) in communication with the image detector (207) disposed at the elongated housing proximal end (204); and
attachment means configured to attach a substantially ring shaped vagina expander (310) to the distal end of the elongated housing,
wherein the connecting portion is configured to convey an image from the image detector to an external device.

2. The intravaginal imaging device of claim 1, wherein the focus mechanism is an autofocus mechanism, and/or
wherein the autofocus mechanism is an autofocus mechanism and further comprises a liquid lens, and/or
wherein the focus mechanism is an autofocus mechanism which has a range of zero to infinity.

3. The intravaginal imaging device of claim 1, further comprising a substantially ring shaped vagina expander disposed at the elongated housing distal end, comprising a substantially concentric aperture and an aperture diameter configured to accommodate an outer diameter of the elongated housing distal end, the vagina expander comprising a substantially circular outer diameter larger than the elongated housing distal end outer diameter.

4. The intravaginal imaging device of claim 3, wherein the vaginal expander is removable, and/or
wherein the vaginal expander outer diameter size is configurably variable.

5. The intravaginal imaging device of claim 4, wherein the configurably variable vaginal expander further comprises an inflatable ring or tube.

6. The intravaginal imaging device of claim 3, wherein the vaginal expander further comprises a mucus trap.

7. The intravaginal imaging device of claim 1, wherein the connecting portion is configured to wirelessly convey the image from the image detector to the external device.

8. The intravaginal imaging device of claim 1, wherein the elongated housing proximal end has a first center axis and the distal end has a second center axis, wherein the first center axis and the second center axis are not parallel.

9. The intravaginal imaging device of claim 1, wherein the elongated housing is hygienically sealed.

10. The intravaginal imaging device of claim 1, further comprising a variable optical aperture (210).

11. The intravaginal imaging device of claim 10, wherein the variable optical aperture further comprises a liquid crystal display.

12. The intravaginal imaging device of claim 1, wherein the light source is disposed behind the window, and/or
wherein the light source comprises a first LED having a first wavelength and a second LED having a second wavelength.

13. The intravaginal imaging device of claim 1, wherein the elongated housing distal end further comprises an optical axis in common with the endcap window, the objective, the focus mechanism, the image detector, and the light source.

14. The intravaginal imaging device of claim 1, further configured to superimpose a scale upon an image collected at the image detector to indicate a size of a region of interest.

15. The intravaginal imaging device of claim 1, further comprising means to attach the elongated housing to a speculum.
